(19) Europäisches Patentamt
European Patent Office
Office européen des brevets

(11) Veröffentlichungsnummer : **0 080 102**
**B1**

(12) **EUROPÄISCHE PATENTSCHRIFT**

(45) Veröffentlichungstag der Patentschrift : **28.05.86**

(51) Int. Cl.⁴ : **C 07 D249/08**, A 01 N 43/653// C07C49/163

(21) Anmeldenummer : **82110296.9**

(22) Anmeldetag : **09.11.82**

(54) **Cycloalkyl-(alpha-triazolyl-beta-hydroxy)-ketone, Verfahren zu ihrer Herstellung sowie ihre Verwendung als Fungizide und Pflanzenwachstumsregulatoren.**

(30) Priorität : **19.11.81 DE 3145846**

(43) Veröffentlichungstag der Anmeldung : **01.06.83 Patentblatt 83/22**

(45) Bekanntmachung des Hinweises auf die Patenterteilung : **28.05.86 Patentblatt 86/22**

(84) Benannte Vertragsstaaten : **AT BE CH DE FR GB IT LI NL SE**

(56) Entgegenhaltungen : **EP-A- 0 007 505**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Kranz, Eckart, Dr.**
**Am Acker 9**
**D-5600 Wuppertal 1 (DE)**
Erfinder : **Büchel, Karl Heinz, Prof. Dr.**
**Dabringhausener Strasse 42**
**D-5093 Burscheid (DE)**
Erfinder : **Kraatz, Udo, Dr.**
**Andreasstrasse 22a**
**D-5090 Leverkusen 1 (DE)**
Erfinder : **Regel, Erik, Ing. grad.**
**Bergerheide 72a**
**D-5600 Wuppertal (DE)**
Erfinder : **Brandes, Wilhelm, Dr.**
**Eichendorffstrasse 3**
**D-5653 Leichlingen 1 (DE)**
Erfinder : **Frohberger, Paul-Ernst, Dr.**
**Willi-Baumeister-Strasse 5**
**D-5090 Leverkusen 1 (DE)**
Erfinder : **Lürssen, Klaus, Dr.**
**August-Kierspel-Strasse 89**
**D-5060 Bergisch Gladbach 2 (DE)**

**EP 0 080 102 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Cycloalkyl-(α-triazolyl-ß-hydroxy)-ketone, ein Verfahren zu ihrer Herstellung sowie ihre Verwendung als fungizide und Pflanzenwachstumsregulatoren.

Es ist bereits bekannt geworden, daß bestimmte Alkyl- bzw. Phenyl-(α-triazolyl-β-hydroxy)-ketone eine gute fungizide Wirksamkeit besitzen (vergleiche DE-A-28 32 233 bzw. EP-A-0 007 505).

So lassen sich zum Beispiel 1,1,1-Trichlor-2-hydroxy-3-(1,2,4-triazol-1-yl)-4-(4-chlorphenyl)-butan-4-on, 2-Chlor-3-hydroxy-2,7,7-trimethyl-4-(1,2,4-triazol-1-yl)-heptan-5-on und 1,1,1-Trichlor-2-hydroxy-5,5-dimethyl-3-(1,2,4-triazol-1-yl)-hexan-4-on zur Bekämpfung von Pilzen einsetzen. Die Wirkung dieser Verbindungen ist jedoch, insbesondere bei niedrigen Aufwandmengen und -konzentrationen, nicht immer voll befriedigend.

Es wurden neue Cycloalkyl-(α-triazolyl-β-hydroxy)-ketone der Formel

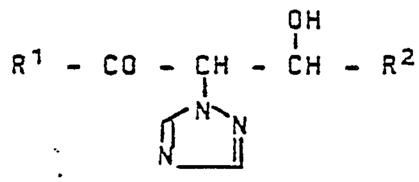

(I)

in welcher

$R^1$ für gegebenenfalls durch Alkyl mit 1 bis 6 Kohlenstoffatomen substituiertes Cycloalkyl mit 3 bis 8 Kohlenstoffatomen steht und

$R^2$ für geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl mit 2 bis 4 Kohlenstoffatomen und 1 bis 5 gleichen oder verschiedenen Halogenatomen, sowie für Alkoxycarbonyl mit 1 bis 4 Kohlenstoffatomen im Alkoxyteil steht,

sowie deren physiologisch verträgliche Säureadditions-Salze und Metallsalz-Komplexe gefunden.

Die erfindungsgemäßen Verbindungen der Formel (I) besitzen zwei asymmetrische Kohlenstoffatome ; sie können deshalb in der erythro- wie in der threo-Form vorliegen. Sie fallen im allgemeinen als Diastereomerengemische unterschiedlicher Zusammensetzung an. In allen Fällen liegen sie vorwiegend als Racemate vor.

Weiterhin wurde gefunden, daß man die Cycloalkyl-(α-triazolyl-β-hydroxy)-ketone der Formel (I) sowie deren physiologisch verträgliche Säureadditions- Salze und Metallsalz-Komplexe erhält, wenn man α-Triazolyl-ketone der Formel

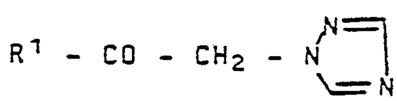

(II)

in welcher $R^1$ die oben angegebene Bedeutung hat, mit Aldehyden der Formel

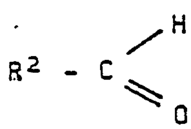

(III)

in welcher $R^2$ die oben angegebene Bedeutung hat, in Gegenwart eines Verdünnungsmittels und in Gegenwart eines Katalysators umsetzt, und gegebenenfalls anschließend in üblicher Weise eine Säure oder ein Metallsalz addiert.

In manchen Fällen erweist es sich als vorteilhaft, die Aldehyde der Formel (III) in Form ihrer Hydrate oder Halbacetale einzusetzen.

Schließlich wurde gefunden, daß die neuen Cycloalkyl-(α-triazolyl-β-hydroxy)-ketone der Formel (I) sowie deren Säureadditons-Salze und Metallsalz-Komplexe starke fungizide und pflanzenwachstumsregulierende Eigenschaften besitzen.

Überraschenderweise zeigen die erfindungsgemäßen Stoffe eine bessere fungizide Wirksamkeit als die aus dem Stand der Technik bekannten Verbindungen 1,1,1-Trichlor-2-hydroxy-3-(1,2,4-triazol-1-yl)-4-(4-chlorphenyl)-butan-4-on, 2-Chlor-3-hydroxy-2,7,7-trimethyl-4-(1,2,4-triazol-1-yl)-heptan-5-on und 1,1,1-Trichlor-2-hydroxy-5,5-dimethyl-3-(1,2,4-triazol-1-yl)-hexan-4-on, welches konstitutionell und wirkungsmäßig ähnliche Stoffe sind. Darüberhinaus ziechnen sich die erfindungsgemäßen Stoffe unerwarteterweise auch durch sehr gute pflanzenwuchsregulierende Eigenschaften aus.

Die erfindungsgemäßen Cycloalkyl-(α-triazolyl-β-hydroxy)-ketone sind durch die Formel (I) allgemein definiert.

Bevorzugt sind diejenigen Verbindungen der Formel (I), in denen

$R^1$ für gegebenenfalls durch Methyl, Ethyl oder Propyl substituiertes Cyclopropyl, Cyclopentyl oder Cyclohexyl steht und

$R^2$ für Trichlormethyl, Dichlorfluormethyl, Trifluormethyl, Dichlormethyl, Chlormethyl, 1,1,2-Trichlorethyl, 1,1-Dichlorethyl, 1,1-Dibromethyl, 1,1-Dichlorpropyl, 1,1-Dichlorbutyl, 1,1,2-Trichlorpropyl, 2-Chlor-prop-2-yl, 1,2,2-Trichlorvinyl, 2,2-Dichlorvinyl, 1,1-Dichlor-2-methyl-propyl, Methoxycarbonyl oder Ethoxycarbonyl steht.

Im einzelnen seien außer den bei den Herstellungsbeispielen angegebenen Verbindungen die folgenden Verbindungen der allgemeinen Formel (I) genannt :

Tabelle 1

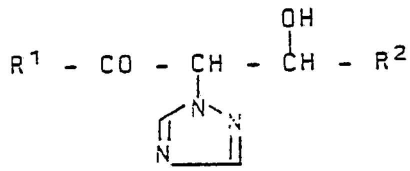

| $R^1$ | $R^2$ |
|---|---|
| 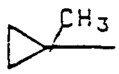 | $-CHCl_2$ |
| 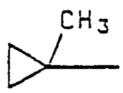 | $-CF_3$ |
| 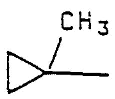 | $-CCl(CH_3)_2$ |
| 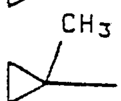 | $-CH=CCl_2$ |
| 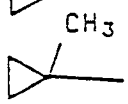 | $-COOCH_3$ |
| 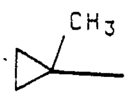 | $-COOC_2H_5$ |
| 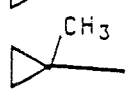 | $-CCl_2CH(CH_3)_2$ |
| 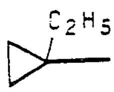 | $-CCl_3$ |
| 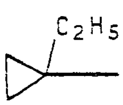 | $-CCl_2CH_2Cl$ |
| 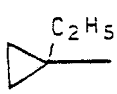 | $-CCl_2CH_3$ |
| 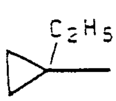 | $-CCl_2CHClCH_3$ |
| 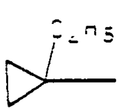 | $-CHCl_2$ |

Tabelle 1 (Fortsetzung)

| $R^1$ | $R^2$ |
|---|---|
| 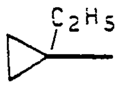 | $-CCl_2CH(CH_3)_2$ |
| 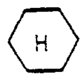 | $-CCl_3$ |
| 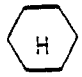 | $-CHCl_2$ |
| 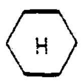 | $-CCl_2CH_3$ |
| 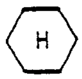 | $-CCl_2CH_2Cl$ |
| 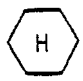 | $-CCl_2CHClCH_3$ |
| 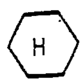 | $-CCl_2CH(CH_3)_2$ |

Bevorzugte erfindungsgemäße Verbindungen sind auch Additionsprodukte aus Säuren und denjenigen Cycloalkyl-(α-triazolyl-β-hydroxy)-ketonen der Formel (I), in denen die Reste $R^1$ und $R^2$ die Bedeutungen haben, die bereits vorzugsweise für diese Reste genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z. B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Cycloalkyl-(α-triazolyl-β-hydroxy)-ketonen der Formel (I), in denen $R^1$ und $R^2$ die Bedeutungen haben, die bereits vorzugsweise für diese Reste genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffe, wie z. B. die Chlorwasserstoffsäuren und die Bromwasserstoffsäure, ferner Phosphorsäure, Salpetersäure und Schwefelsäure.

Verwendet man beispielsweise 1-Methyl-1-(1,2,4-triazol-1-yl-acetyl)-cyclopropan und Chloral als Ausgangsstoffe, so kann der Reaktionsablauf nach dem erfindungsgemäßen Verfahren durch das folgende Formelschema wiedergegeben werden :

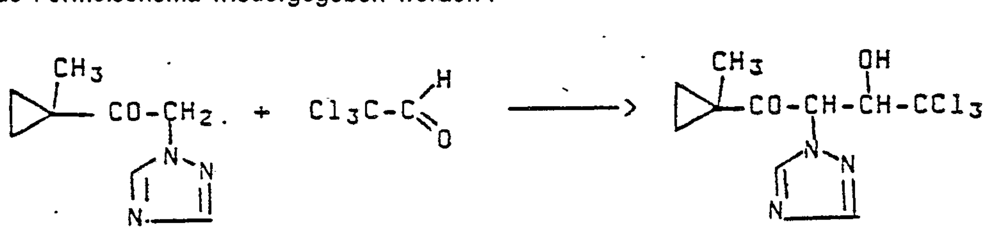

Die für das erfindungsgemäße Verfahren als Ausgangsstoffe zu verwendenden α-Triazolyl-ketone sind durch die Formel (II) allgemein definiert. In dieser Formel steht $R^1$ vorzugsweise für diejenigen Bedeutungen, edie bereits im Zusammenhang mit der Beschreibung der erfindungsgemäßen Stoffe der Formel (I) als bevorzugt für diesen Substituenten genannt wurden.

Die α-Triazolyl-ketone der Formel (II) sind teilweise bekannt (vgl. DE-A-24 31 407 und DE-A-30 10 560).

Die bisher noch nicht bekannten α-Triazolyl-ketone sind definiert durch die Formel

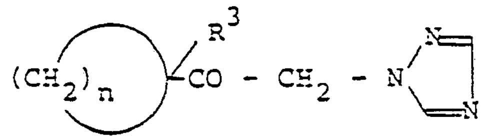

(IIa)

in welcher
$R^3$ für Alkyl mit 1 bis 6 Kohlenstoffatomen, steht und
n für ganze Zahlen von 3 bis 7 steht.

Die bisher noch nicht bekannten α-Triazolyl-ketone der Formel (IIa), die Gegenstand eines gesonderten Schutzbegehrens sind, lassen sich herstellen, indem man Halogenverbindungen der Formel

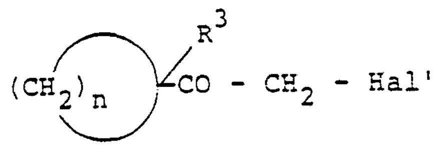

(IV)

in welcher
$R^3$ und n die oben angegebene Bedeutung haben und
Hal' für Chlor oder Brom steht,
mit 1,2,4-Triazol der Formel

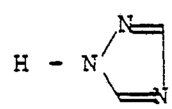

(V)

in Gegenwart eines inerten organischen Lösungsmittels sowie in Gegenwart eines Säurebindemittels umsetzt.

Die bei der obigen Umsetzung zur Herstellung der α-Triazolyl-ketone der Formel (IIa) als Ausgangsstoffe benötigten Halogenverbindungen der Formel (IV) sind bisher noch nicht bekannt. Sie lassen sich jedoch nach im Prinzip bekannten Verfahren in einfacher Weise herstellen.

So erhält man die Halogenverbindungen der Formel (IV) zum Beispiel dadurch, daß man Ketone der Formel

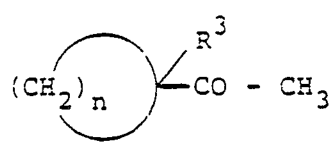

(VI)

in welcher $R^3$ und n die oben angegebene Bedeutung haben, in Gegenwart eines inerten organischen Lösungsmittels bei Raumtemperatur mit Chlor oder Brom versetzt ; oder z. B. mit üblichen Chlorierungsmitteln, wie Sulfurylchlorid, bei 20 bis 60 °C umsetzt.

Die Ketone der Formel (VI) werden erhalten, indem man Nitrile der Formel

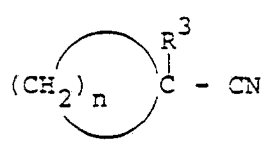

(VII)

in welcher $R^3$ und n die oben angegebene Bedeutung haben, in üblicher Weise mit metallorganischen Verbindungen, wie insbesondere Methylmagnesiumbromid, in Gegenwart eines Verdünnungsmittels, wie z. B. wasserfreie Ether, bei Temperaturen zwischen 0 und 80 °C umsetzt.

Die Nitrile der Formel (VII) sind bekannt (vgl. Journal of Organometallic Chemistry *57*, C 33-35 (1973)) bzw. können sie nach dem dort angegebenen Verfahren erhalten werden.

Als Verdünnungsmittel kommen bei der Herstellung der α-Triazolyl-ketone der Formel (IIa) nach dem obigen Verfahren alle inerten organischen Solventien in Frage. Vorzugsweise in Betracht kommen Nitrile, wie Acetonitril, weiterhin Ketone, wie Aceton und Methyl-n-butyl-keton, ferner Alkohole, wie Ethanol, Propanol und n-Butanol, außerdem aromatische Kohlenwasserstoffe, wie Toluol, und auch polare Solventien, wie Dimethylformamid und Dimethylsulfoxid.

Als Säurebindemittel können bei der oben beschriebenen Herstellung der α-Triazolyl-ketone der Formel (IIa) alle üblicherweise für derartige Umsetzungen geeigneten anorganischen und organischen Basen eingesetzt werden. Vorzugsweise verwendbar sind Alkalicarbonate, wie Natriumund Kaliumcarbonat oder Natriumhydrogencarbonat, ferner niedere tertiäre Alkylamine, Aralkylamine, aromatische Amine oder Cycloalkylamine, wie zum Beispiel Triethylamin, Dimethylbenzylamin, Pyridin, 1,5-Diaza-bicyclo [4,3,0] non-5-en (DBN) und 1,8-Diaza-bicyclo [5,4,0]-undec-7-en (DBU). Es ist aber auch möglich, einen entsprechenden Überschuß an 1,2,4-Triazol einzusetzen.

Die Temperaturen lassen sich bei dem obigen Verfahren zur Herstellung der α-Triazolyl-ketone der Formel (IIa) innerhalb eines größeren Bereiches variieren. Im allgemeinen arbeitet man bei Temperaturen zwischen 20 °C und 120 °C, vorzugsweise zwischen 40 °C und 90 °C.

Bei der Durchführung des obigen Verfahrens zur Herstellung der α-Triazolyl-ketone der Formel (IIa) setzt man im allgemeinen auf 1 Mol an Halogenverbindung der Formel (IV) 1 Mol oder auch einen Überschuß an 1,2,4-Triazol der Formel (V) ein. Die Aufarbeitung erfolgt nach üblichen Methoden.

Die übrigen Verbindungen der Formel (II) lassen sich ebenso herstellen, wie es für die α-Triazolyl-ketone der Formel (IIa) beschrieben wurde.

Die außerdem für die erfindungsgemäße Umsetzung als Ausgangsstoffe zu verwendenden Aldehyde sind durch die Formel (III) allgemein definiert. In dieser Formel steht $R^2$ vorzugsweise für diejenigen Bedeutungen, die bereits im Zusammenhang mit der Beschreibung der erfindungsgemäßen Stoffe der Formel (I) als bevorzugt für diesen Substituenten genannt wurden.

Die Aldehyde der Formel (III) sind allgemein bekannte Verbindungen der organischen Chemie.

Als Verdünnungsmittel für die erfindungsgemäße Umsetzung kommen vorzugsweise inerte orgarische Lösungsmittel infrage. Hierzu gehören vorzugsweise Alkohole, wie Methanol und Ethanol, sowie deren Mischungen mit Wasser ; Ether, wie Tetrahydrofuran und Dioxan ; Nitrile, wie Acetonitril und Propionitril ; halogenierte aliphatische und aromatische Kohlenwasserstoffe, wie Methylenchlorid, Tetrachlorkohlenstoff, Chloroform, Chlorbenzol und Dichlorbenzol ; sowie Eisessig.

Die erfindungsgemäße Umsetzung wird in Gegenwart eines Katalysators vorgenommen. Man kann alle üblicherweise verwendbaren sauren und insbesondere basischen Katalysatoren einsetzen. Hierzu gehören vorzugsweise Lewis-Säuren, wie z. B. Eisen-(III)-chlorid, Eisen-(III)-bromid, Bortrifluorid, Bortrichlorid, Zinntetrachlorid oder Titantetrachlorid ; ferner Alkali- und Erdalkalihydroxide, wie Kaliumhydroxid, Natriumhydroxid, Calciumhydroxid oder Bariumhydroxid ; außerdem Alkalisalze, wie Kaliumcarbonat, Natriumcarbonat, Kaliumcyanid, sekundäres Natriumphosphat, Natriumacetat und Natriumsulfit ; sowie Alkoholate, wie Natrium- oder Kaliummethylat.

Die Reaktionstemperaturen können bei der Durchführung des erfindungsgemäßen Verfahrens in einem größeren Bereich variiert werden. Im allgemeinen arbeitet man bei Temperaturen zwischen 0 °C und 100 °C, vorzugsweise bei Raumtemperatur bzw. dem Siedepunkt des jeweiligen Lösungsmittels.

Bei der Durchführung des erfindungsgemäßen Verfahrens setzt man die Reaktionskomponenten der Formeln (II) und (III) in allgemeinen in äquimolaren Mengen ein. Außerdem fügt man eine katalytische oder auch äquimolare Menge an Katalysator hinzu. Es ist auch möglich, eine der Reaktionskomponenten der Formeln (II) und (III) in einem Überschuß einzusetzen. Die Isolierung der Verbindungen der Formel (I) erfolgt in üblicher Weise (vergleiche auch die Herstellungsbeispiele).

Die nach dem erfindungsgemäßen Verfahren herstellbaren Cycloalkyl-(α-triazolyl-β-hydroxy)-ketone der Formel (I) können in Säureadditions-Salze beziehungsweise Metallsalz-Komplexe überführt werden.

Zur Herstellung von physiologisch verträglichen Säure-additions-Salzen der Stoffe der Formel (I) kommen vorzugsweise diejenigen Säuren in Frage, die bereits im Zusammenhang mit der Beschreibung der bevorzugten Säureadditions-Salze der Verbindungen der Formel (I) genannt wurden.

Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejeingen Metallsalze in Frage, die bereits im Zusammenhang mit der Beschreibung der bevorzugten Metallsalz-Komplexe der Verbindungen der Formel (I) genannt wurden.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, Isolieren und gegebenenfalls durch Umkristallisieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindugsgemäßen Wirkstoffe mit besonders gutem Erfolg zur Bekämfung solcher Pilze eingesetzt werden, die echte Mehltauerkrankungen hervorrufen, so zur Bekämpfung des Gurkenmehltaus (Sphaerotheca fuligina) und des Gerstenmehltaus (Erysiphe graminis), sowie zur Bekämpfung von Venturia-Arten, wie gegen den Erreger des Apfelschorfs (Venturia inaequalis), und von Reiskrankheiten, wie Pyricularia oryzae.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachtstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuches von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (« Lagerns ») der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte enstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingelhalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (« Ausdünnung »), um die Alternanz zu brechen. Unter Alternanz versteht man die Eigenart einiger Obstarten, endogen bedingt von Jahr zu Jahr

sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z. B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z. B. Ananas oder Zierpflanzen in Gärtnereien, zu einem Zeitpunkt keinem, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage : z. B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arysulfonate sowie Eiweißhydrolysate. Als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen Schäume, Suspensionen, Spritzpulver, Pasten, lösliche Pulver, Stäubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar

Bodenfläche 0,01 bis 50 kg bevorzugt 0,05 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach klimatischen und vegetativen Gegebenheiten richtet.

Auch beim Einsatz der erfindugsgemäßen Stoffe als Fungizide kann die Aufwandmenge je nach Art der Applikation in einem größeren Bereich variiert werden. So liegen die Wirkungskonzentrationen bei der Behandlung von Pflanzenteilen in den Anwendungsformen im allgemeinen zwischen 1 und 0,000 1 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei der Behandlung des Bodens sind Wirkstoffkonzentrationen von 0,000 1 bis 0,1 Gew.-%, vorzugsweise von 0,000 1 bis 0,02 %, am Wirkungsort erforderlich.

Die Herstellung und die Verwendung der erfindungsgemäßen Wirkstoffe werden durch die nachfolgenden Beispiele veranschaulicht.

## Herstellungsbeispiele

### Beispiel 1

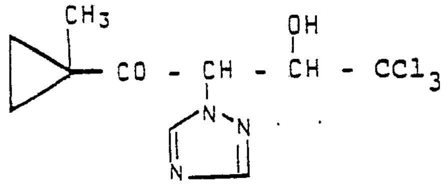

Zu 8,3 g (0,05 Mol) 1-Methyl-1-(1,2,4-triazol-1-yl-acetyl)-cyclopropan, 20 ml Eisessig und 13,6 g (0,1 Mol) Natriumacetat werden 9,2 g (0,062 5 Mol) Chloral getropft, wobei die Innentemperatur auf ca. 45 °C ansteigt. Man läßt das Reaktionsgemisch bei 80 °C über Nacht rühren, danach auf Raumtemperatur abkühlen und verrühlen und verrührt mit einem Gemisch aus 100 ml Wasser und 100 ml Ether. Der entstehende Feststoff wird abgesaugt und aus Methanol umkristallisiert. Man erhält 3,1 g (20 % der Theorie) 1,1,1-Trichlor-2-hydroxy-3-(1,2,4-triazol-1-yl)-4-(1-methylcyclopropan-1-yl)-butan-4-on vom Schmelzpunkt 216-18 °C.

Herstellung des Ausgangsproduktes

a)

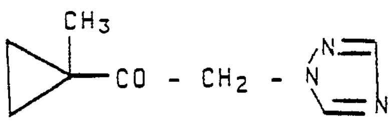

In eine Suspension von 27,6 g (0,4 Mol) 1,2,4-Triazol und 41,4 g (0,3 Mol) Kaliumcarbonat in 500 ml Aceton werden bei 60 °C 42,5 g (0,24 Mol) 1-Bromacetyl-1-methyl-cyclopropan eingetropft. Nach 15 Stunden Erhitzen auf 60 °C werden die Salze abgesaugt und das Filtrat wird im Vakuum eingedampft. Das verbleibende Oel wird chromatographisch (Kieselgel-60 (Merck)/Chloroform) gereinigt.

Man erhält 35,7 g (90 % der Theorie) 1-Methyl-1-(1,2,4-triazol-1-yl-acetyl)yclopropan vom Schmelzpunkt 58 °C.

b)

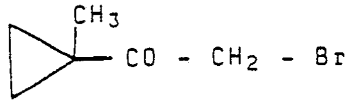

Zu einer Lösung von 29,4 g (0,3 Mol) 1-Acetyl-1-methyl-1-cyclopropan in 150 ml Methylalkohol werden bei 5 °C 15 ml Brom, gelöst in 75 ml Chloroform, getropft. Die Lösung wird bei 10 °C bis zur vollständigen Entfärbung gerührt und auf Eis gegeben. Die organische Phase wird mit Wasser gewaschen, über Natriumsulfat getrocknet, filtriert, eingedampft und destilliert. Man erhält 44 g (82,5 % der Theorie) 1-Bromacetyl-1-methylcyclopropan vom Siedepunkt 85 bis 90 °C/11 Torr bzw. vom Brechungsindex $n_D^{20}$ = 1,500 2.

Beispiel 2

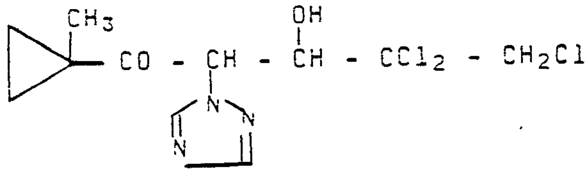

Zu 9,2 g (0,056 Mol) 1-Methyl-1-(1,2,4-triazol-1-yl-acetyl)-cyclopropan in 120 ml Tetrahydrofuran und 13,6 g (0,1 Mol) Kaliumcarbonat werden 11,3 g 2,2,3-Trichlorpropionaldehyd getropft, wobei die Temperatur leicht ansteigt. Man läßt über Nacht bei Raumtemperatur rühren und gibt die Reaktionslösung danach in 500 ml Wasser. Der entstehende Niederschlag wird abgesaugt und aus Methanol umkristallisiert. Man erhält 5,5 g (30 % der Theorie) 1,2,2-Trichlor-3-hydroxy-4-(1,2,4-triazol-1-yl)-5-(1-methylcycloprop-1-yl)-pentan-5-on vom Schmelzpunkt 182-86 °C.

Beispiel 3

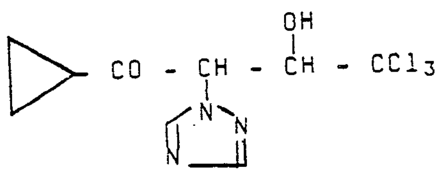

Zu 7,6 g (0,05 Mol) 1,2,4-Triazol-1-yl-acetyl-cyclopropan, 7,5 g Eisessig und 4,1 g (0,05 Mol) Natriumacetat werden 14,8 g (0,1 Mol) Chloral getropft, wobei die Innentemperatur auf ca. 42 °C ansteigt. Man läßt das Reaktionsgemisch 5 Stunden bei 80 °C und 12 Stunden bei Raumtemperatur rühren und gibt es dann auf Wasser. Der entstehende Niederschlag wird abgesauft und aus Toluol umkristallisiert. Man erhält 8,6 g (57,6 % der Theorie) 1,1,1-Trichlor-2-hydroxy-3-(1,2,4-triazol-1-yl)-4-cyclopropyl-butan-4-on vom Schmelzpunkt 173-75 °C.

In entsprechender Weise werden die folgenden in Tabelle 2 aufgeführten Verbindungen der Formel

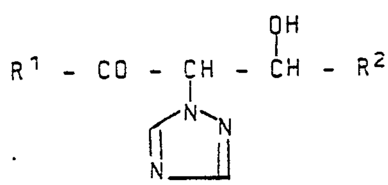

(I)

erhalten :

Tabelle 2

| Bsp. Nr. | $R^1$ | $R^2$ | Schmelzpunkt (°C) |
|---|---|---|---|
| 4 | 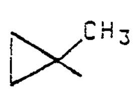 | $-CCl_2CH_3$ | 196-98 |
| 5 | 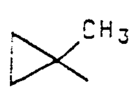 | $-CCl_2-C_2H_5$ | 158-61 |
| 6 | 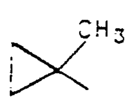 | $-CCl_2-C_3H_7-i$ | 134-37 |
| 7 | 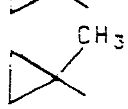 | $-CCl_2-CHCl-CH_3$ | 178-79 |
| 8 | 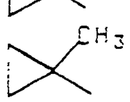 | $-CCl_2-C_3H_7-n$ | 161-62 |

(Fortsetzung)

| Bsp. Nr. | $R^1$ | $R^2$ | Schmelzpunkt (°C) |
| --- | --- | --- | --- |
| 9 | 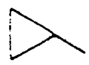 | $-CCl_2-CHCl-CH_3$ | 56-62 |
| 10 | 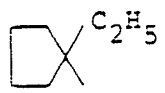 | $-CCl_3$ | 194-196 |
| 11 | 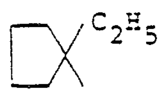 | $-CCl_2-CH_2Cl$ | 185-187 |

## Anwendungsbeispiele

In den nachfolgenden Anwendungsbeispielen werden die nachstehend angegebenen Verbindungen als Vergleichssubstanzen eingesetzt :

(A)

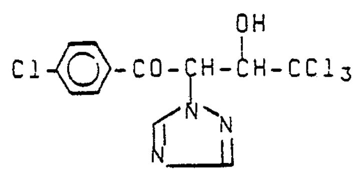

(B)

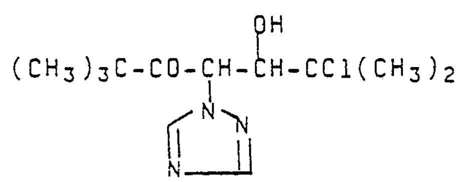

(C)

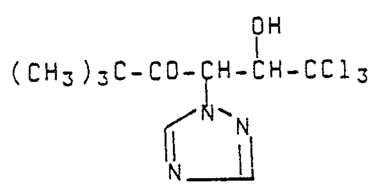

## Beispiel A

Venturia-Test (Apfel)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 1 und 2.

## Beispiel B

Sphaerotheca-Test (Gurke)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 2.

## Beispiel C

Erysiphe-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 1, 2, 3 und 4.

## Beispiel D

Erysiphe-Test (Gerste)/Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 × 12 Korn 2 cm tief in eine Standarderde. 7 Tage nach der Aussaat, wenn die jungen Pflanzen ihr erstes Blatt entfaltet haben, werden sie mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 2.

## Beispiel E

Wuchshemmung bei Grass (Festuca pratensis)

Lösungsmittel : 30 Gewichtsteile Diemthylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnass besprüht. Nach 3 Wochen wird der Zuwachs gemessen und die Wuchshemmung in Prozent des Zuwachses der Kontrollpflanzen berechnet. Es bedeuten 100 % Wuchshemmung den Stillstand des Wachstumes und 0 % ein Wachstum entsprechend dem der Kontrollpflanzen.

In diesem Test zeigt die Verbindung gemäß Beispiel (3) eine starke Wuchshemmung.

Beispiel F

Wuchshemmung bei Gerste

Lösungsmittel : 30 Gewichtsteile Dimethylformamid
Emulgator : 1 Gewichtsteil Polyoxyethylen-Sorbitan-monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstenpflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

In diesem Test zeigt die Verbindung gemäß Beispiel (3) eine starke wuchshemmende Wirkung.

Beispiel G

Wuchsbeeinflussung bei Zuckerrüben

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zurckerrüben werden im Gewächshaus bis zur vollen Ausbildung der Keimblätter angezogen. In diesem Stadium werden die Pflanzen tropfnaß mit den Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

In diesem Test zeigt die Verbindung gemäß Beispiel (3) eine starke wuchshemmende Wirkung.

**Patentansprüche**

1. Cycloalkyl-(α-triazolyl-β-hydroxy)-ketone der Formel

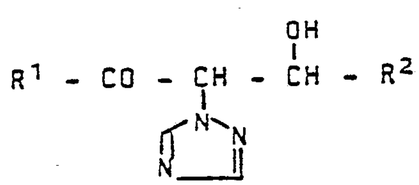

(I)

in welcher

$R^1$ für gegebenenfalls durch Alkyl mit 1 bis 6 Kohlenstoffatomen substituiertes Cycloalkyl mit 3 bis 8 Kohlenstoffatomen steht und

$R^2$ für geradkettiges oder verzweigtes Halogenalkyl mit 1 bis 4 Kohlenstofatomen und 1 bis 5 gleichen oder verschiedenen Halogenatomen, geradkettiges oder verzweigtes Halogenalkenyl mit 2 bis 4 Kohlenstoffatomen und 1 bis 5 gleichen oder verschiedenen Halogenatomen, sowie für Alkoxycarbonyl mit 1 bis 4 Kohlenstoffatomen im Alkoxyteil steht,

sowie deren physiologisch verträgliche Säureadditions-Salze und Metallsalz-Komplexe.

2. Verfahren zur Herstellung von Cycloalkyl-(α-triazolyl-β-hydroxy)-ketonen der Formel

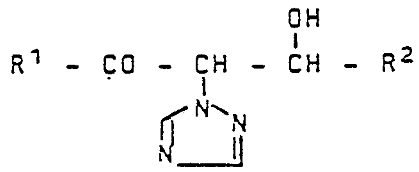

(I)

in welcher

$R^1$ für gegebenenfalls durch Alkyl mit 1 bis 6 Kohlenstoffatomen substituiertes Cycloalkyl mit 3 bis 8 Kohlenstoffatomen steht und

$R^2$ für geradkettiges oder verzweigtes Halogenalkyl mit 1 bis 4 Kohlenstoffatomen und 1 bis 5 gleichen oder verschiedenen Halogenatomen, geradkettiges oder verzweigtes Halogenalkenyl mit 2 bis 4 Kohlenstoffatomen und 1 bis 5 gleichen oder verschiedenen Halogenatomen, sowie für Alkoxycarbonyl mit 1 bis 4 Kohlenstoffatomen im Alkoxyteil steht,

sowie deren physiologisch verträglichen Säureadditions-Salzens und Metallsalz-Komplexen, dadurch gekennzeichnet, daß man α-Triazolyl-ketone der Formel

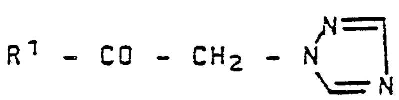

(II)

in welcher $R^1$ die oben angegebene Bedeutung hat, mit Aldehyden der Formel

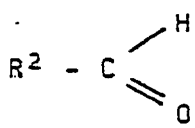

(III)

in welcher $R^2$ die oben angegebene Bedeutung hat, in Gegenwart eines Verdünnungsmittels und in Gegenwart eines Katalysators umsetzt, und gegebenenfalls anschließend in üblicher Weise eine Säure oder ein Metallsalz addiert.

3. Fungizide und pflanzenwuchsregulierende Mittel, gekennzeichnet durch einen Gehalt an mindestens einem Cycloalkyl-(α-triazolyl-β-hydroxy)-keton der Formel (I) gemäß Anspruch 1 bzw. einem physiologisch verträglichen Säureadditions-Salz oder Metallsalz-Komplex von einem Cycloalkyl-(α-triazolyl-β-hydroxy)-keton der Formel (I) gemäß Anspruch 1.

4. Verwendung von Cycloalkyl-(α-triazolyl-β-hydroxy)-ketonen der Formel (I) gemäß Anspruch 1 bzw. deren physiologisch verträglichen Säureadditions-Salzen und Metallsalz-Komplexen zur Bekämpfung von Pilzen bzw. zur Regulierung des Pflanzenwachstums.

5. Verfahren zur Herstellung von fungiziden und pflanzenwuchsregulierenden Mitteln, dadurch gekennzeichnet, daß man C cloalkyl-(α-triazolyl-β-hydrox)-ketone der Formel (I) gemäß Anspruch 1 bzw. deren physiologisch verträgliche Säureadditions-Salze oder Metallsalz-Komplexe mit Streckmitteln und/oder oberflächenaktiven Stoffen vermischt.

## Claims

1. Cycloalkyl-(α-triazolyl-β-hydroxy) ketones of the formula

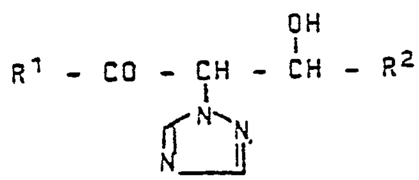

(I)

in which

$R^1$ represents cycloalkyl which has 3 to 8 carbon atoms and its optionally substituted by alkyl with 1 to 6 carbon atoms and

$R^2$ represents straight-chain or branched halogenoalkyl with 1 to 4 carbon atoms and 1 to 5 identical

or different halogen atoms, straight-chain or branched halogenoalkenyl with 2 to 4 carbon atoms and 1 to 5 identical or different halogen atoms, and alkoxycarbonyl with 1 to 4 carbon atoms in the alkoxy part, and physiologically tolerated acid addition salts and metal salt complexes thereof.

2. Process for the preparation of cycloalkyl-(α-triazolyl-β-hydroxy)-ketones of the formula

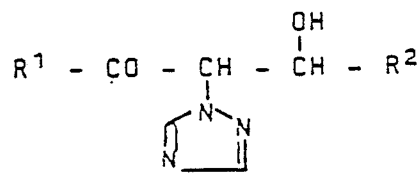

(I)

in which

$R^1$ represents cycloalkyl which has 3 to 8 carbon atoms and is optionally substituted by alkyl with 1 to 6 carbon atoms and

$R^2$ represents straight-chain or branched halogenoalkyl with 1 to 4 carbon atoms and 1 to 5 identical or different halogen atoms, straight-chain or branched halogenoalkenyl with 2 to 4 carbon atoms and 1 to 5 identical or different halogen atoms, and alkoxycarbonyl with 1 to 4 carbon atoms in the alkoxy part, and physiologically tolerated acid addition salts and metal salt complexes thereof, characterised in that α-triazolyl-ketones of the formula

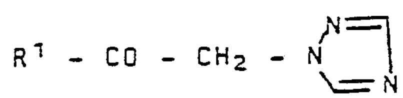

(II)

in which $R^1$ has the abovementioned meaning, are reacted with aldehydes of the formula

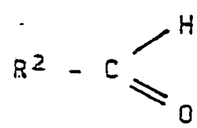

(III)

in which $R^2$ has the abovementioned meaning, in the presence of a diluent and in the presence of a catalyst, and, if desired, an acid or a metal salt is then added on in a customary manner.

3. Fungicidal and plant-growth-regulating agents, characterised in that they contain at least one cycloalkyl-(α-triazolyl-β-hydroxy)-ketone of the formula (I) according to Claim 1 or at least one physiologically tolerated acid addition salt or metal salt complex of a cycloalkyl-(α-triazolyl-β-hydroxy)-ketone of the formula (I) according to Claim 1.

4. Use of cycloalkyl-(α-triazolyl-β-hydroxy)-ketones of the formula (I) according to Claim 1 or physiologically tolerated acid addition salts and metal salt complexes thereof, for combating fungi or for regulating plant growth.

5. Process for the preparation of fungicidal and plant-growth-regulating agents, characterised in that cycloalkyl-(α-triazolyl-β-hydroxy)-ketones of the formula (I) according to Claim 1 or physiologically tolerated acid addition salts or metal salt complexes thereof are mixed with extenders and/or surface-active substances.

**Revendications**

1. Cycloalkyl-(α-triazolyl-β-hydroxy)-cétones de formule :

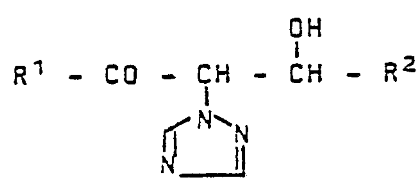

(I)

dans laquelle

$R^1$ représente un groupe cycloalkyle ayant 3 à 8 atomes de carbone et qui est éventuellement substitué par un groupe alkyle ayant 1 à 6 atomes de carbone, et

$R^2$ représente un groupe halogénoalkyle, linéaire ou ramifié, ayant 1 à 4 atomes de carbone et 1 à 5 atomes d'halogène identiques ou différents, un groupe halogéno-alcényle linéaire ou ramifié ayant 2 à 4 atomes de carbone et 1 à 5 atomes d'halogène identiques ou différents, ainsi qu'un groupe alcoxycarbonyle ayant 1 à 4 atomes de carbone dans la partie alcoxy,

ainsi que leurs sels d'addition d'acides et complexes avec des sels de métaux, physiologiquement compatibles.

2. Procédé de préparation de cycloalkyl-(α-triazolyl-β-hydroxy)-cétones de formule :

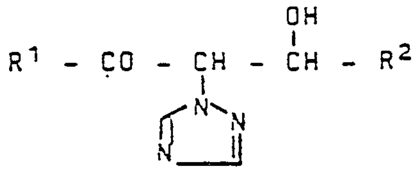

(I)

dans laquelle

$R^1$ représente un groupe cycloalkyle ayant 3 à 8 atomes de carbone et qui est éventuellement substitué par un groupe alkyle ayant 1 à 6 atomes de carbone, et

$R^2$ représente un groupe halogénoalkyle linéaire ou ramifié ayant 1 à 4 atomes de carbone et 1 à 5 atomes d'halogène identiques ou différents, un groupe halogénoalcényle linéaire ou ramifié ayant 2 à 4 atomes de carbone et 1 à 5 atomes d'halogène identiques ou différents, ainsi qu'un groupe alcoxycarbonyle ayant 1 à 4 atomes de carbone dans la partie alcoxy,

ainsi que de leurs sels d'addition d'acides et complexes avec des sels de métaux, physiologiquement compatibles, caractérisé en ce qu'on fait réagir des α-triazolyl-cétones de formule :

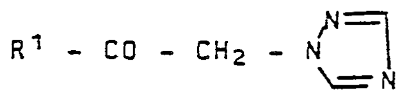

(II)

dans laquelle $R^1$ a le sens indiqué ci-dessus, avec des aldéhydes de formule :

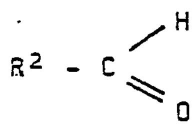

(III)

dans laquelle $R^2$ a le sens indiqué ci-dessus, en présence d'un diluant et en présence d'un catalyseur, et l'on fixe éventuellement ensuite par addition, de façon usuelle, un acide ou un sel de métal.

3. Produit fongicide et à rôle de substance de croissance, caractérisé par une teneur en au moins une cycloalkyl-(α-triazolyl-β-hydroxy)-cétone de formule (I) selon la revendication 1 ou en un sel d'addition d'acide ou complexe avec sel de métal, physiologiquement compatible, d'une cycloalkyl-(α-triazolyl-β-hydroxy)-cétone de formule (I) selon la revendication 1.

4. Utilisation de cycloalkyl-(α-triazolyl-β-hydroxy)-cétones de formule (I) selon la revendication 1 ou de leurs sels d'addition d'acides et leurs complexes avec des sels de métaux, physiologiquement compatibles, pour combattre des champignons ou pour réguler la croissance de plantes.

5. Procédé de préparation de fongicides et de produits à rôle de substances de croissance, caractérisé en ce qu'on mélange, avec des agents d'allongement et/ou des substances tensioactives, des cycloalkyl-(α-triazolyl-β-hydroxy)-cétones de formule (I) selon la revendication 1 ou leurs sels d'addition d'acides ou leurs complexes avec des sels de métaux, physiologiquement compatibles.